# EUROPÄISCHE PATENTSCHRIFT

(11) **EP 3 076 851 B1**
(45) Veröffentlichungstag und Bekanntmachung des Hinweises auf die Patenterteilung: **03.08.2022**
(21) Anmeldenummer: 14806228.4
(22) Anmeldetag: 28.11.2014
(51) Int. Cl.: A61B 1/00, A61B 1/005

(54) **ENDOSKOPKOPF UND ENDOSKOP**
ENDOSCOPE HEAD AND ENDOSCOPE
TÊTE D'ENDOSCOPE ET ENDOSCOPE

(30) Priorität: 02.12.2013 DE 102013224683
(43) Veröffentlichungstag der Anmeldung: 12.10.2016
(73) Patentinhaber: Digital Endoscopy GmbH, 86316 Friedberg (DE)
(72) Erfinder: VIEBACH, Thomas, 86579 Waidhofen (DE); PAUKER, Fritz, 86420 Diedorf (DE)
(74) Vertreter: TBK
(86) Internationale Anmeldenummer: PCT/EP2014/075902
(87) Internationale Veröffentlichungsnummer: WO 2015/082328

(56) Entgegenhaltungen:
- EP-A1- 1 030 607
- WO-A1-2013/129204
- JP-A- H11 244 225

## Beschreibung

Die vorliegende Erfindung bezieht sich auf einen Endoskopkopf und auf ein mit diesem versehenes Endoskop. Genauer gesagt bezieht sich die vorliegende Erfindung auf einen verbesserten Endoskopkopf, der am distalen Ende eines Deflectingabschnittes eines Endoskops einsetzbar ist.

### Ausgangspunkt der Erfindung

In der Endoskopie geht der Trend seit geraumer Zeit hin zu immer kleineren Endoskopen. In diesem Zusammenhang wird auch ein Endoskopkopf, der am distalen Ende eines Deflectingabschnittes eines Endoskops angeordnet ist, immer kleiner gestaltet. Dabei wird es immer schwieriger, die in der Endoskopie genutzten Einrichtungen wie z.B. LED, Kamera und/oder Arbeitskanal etc. im Endoskopkopf zu integrieren.

Die WO 2013/129 204 A1 offenbart einen Endoskopabschnitt an einem Teil eines Endoskops. Der Endoskopabschnitt hat Leiterbahnen, die über Kabel mit Strom versorgt werden können.

Die EP 1 030 607 A1 offenbart ein Endoskop mit einem Zugseil zum Handhaben von abzutrennenden Gewebeteilen durch Ziehen und Transportieren von Energie.

Die vorliegende Erfindung soll einen neuen Weg aufzeigen, wie durch innovative Gestaltung eines Endoskopkopfes eine weitere Miniaturisierung von Endoskopen und Endoskopköpfen möglich wird.

### Aufgabe der Erfindung

Somit ist es eine Aufgabe der vorliegenden Erfindung, einen verbesserten Endoskopkopf zu schaffen. Darüber hinaus soll ein verbessertes Endoskop geschaffen werden.

### Lösung

Im Hinblick auf den Endoskopkopf ist die Aufgabe durch einen Endoskopkopf gemäß Anspruch 1 gelöst.

Im Hinblick auf das Endoskop ist die Aufgabe durch ein Endoskop gemäß Anspruch 5 gelöst. Vorteilhafte Weiterbildungen sind Gegenstand der abhängigen Ansprüche.

Die vorliegende Offenbarung betrifft einen Endoskopkopf an einem Deflectingende eines Endoskops, mit einem Endoskopkopfkörper mit zumindest einer an diesem aufgetragenen Leiterbahn; zumindest einem elektronischen Instrument, das im Endoskopkopfkörper sitzt und durch dessen zumindest eine Leiterbahn elektrisch versorgbar ist; und zumindest einem Zugseil, dessen Zugseilverankerung im Endoskopkopfkörper sitzt; wobei das zumindest eine Zugseil mit der zumindest einen Leiterbahn des Endoskopkopfkörpers elektrisch in Verbindung steht.

Dadurch ergibt sich eine kostengünstig gestaltete elektrische Versorgung des Endoskopkopfes bei platzsparender Ausführung. Auf ein Raum benötigendes elektrisches Versorgungskabel für das elektronische Instrument kann verzichtet werden.

Im Endoskopkopf kann das zumindest eine Zugseil über seine Zugseilverankerung mit der zumindest einen Leiterbahn des Endoskopkopfkörpers elektrisch in Verbindung stehen. Die Zugseilverankerung kann ein elektrisch leitfähiger Körper sein, der die Leiterbahn und das Zugseil elektrisch verbindet.

Im Endoskopkopf können am Endoskopkopfkörper vier Zugseile verankert sein, von denen zwei Zugseile mit der zumindest einen Leiterbahn des Endoskopkopfkörpers elektrisch in Verbindung stehen. Von den vier Zugseilen können zwei Zugseile elektrisch leitfähig sein. Die Funktionsweise ist die gleiche wie bei einem herkömmlichen Endoskopkopf, bei dem mittels vier Zugseilen die Auslenkbewegung gesteuert wird. Es können auch alle vier Zugseile elektrisch leitfähig sein.

Die Anzahl an Zugseilen ist nicht eingeschränkt.

Der Endoskopkopfkörper kann ein MID-Formelement sein.

Der Endoskopkopfkörper ist dadurch kostengünstig und umweltverträglich herstellbar, kann vielseitig und mit hoher Gestaltungsfreiheit geformt werden. Beliebige Endoskopkopfkörperformen sind daher möglich. Eine weitere Miniaturisierung der Endoskopkopfe wird verwirklicht. Die Anzahl an verwendeten Materialen zur Herstellung des Endoskopkopfkörpers ist auf ein Minimum beschränkt, was zu einer Material- und Teilezahleinsparung führt. Die Anzahl an Montageschritten wird verringert. Die Genauigkeit der Formgebung und damit auch die Zuverlässigkeit des Endoskopkopfes werden drastisch erhöht. Für den Endoskopkopfkörper können im Gegensatz zu herkömmlichen Leiterplatten unproblematisch entsorgbare Materialien eingesetzt werden. Die axiale Länge des Endoskopkopfkörpers kann verkürzt werden.

Nachstehend sind Ausführungsbeispiele der Erfindung detailliert anhand von Beispielen erläutert.

### Kurzbeschreibung der Zeichnungen

Figur 1 zeigt einen Endoskopkopf mit Zugseilen gemäß einem Ausführungsbeispiel der Erfindung, wobei Fig. 1(A) eine schematische perspektivische Ansicht,
Fig. 1(B) eine Seitenansicht und Fig. 1(C) eine Schnittansicht entlang einer Linie A-A in Fig. 1(B) zeigen.
Figur 2 zeigt den Endoskopkopf mit Zugseilen gemäß dem Ausführungsbeispiel von Figur 1, wobei Fig. 2(A) eine Seitenansicht und Fig. 2(B) eine Schnittansicht entlang einer Linie D-D in Fig. 2(A) zeigen.
Figur 3 zeigt einen Endoskopkopfkörper für den Endoskopkopf gemäß dem Ausführungsbeispiel von Figur 1.
Figur 4 zeigt einen Endoskopkopfkörper von Figur 3, in den Zugseile eingehängt sind.
Figur 5 zeigt einen Endoskopkopfkörper von Figur 4, in den ein Kameramodul und LED-Chips eingesetzt ist.

Nachstehend ist die vorliegenden Erfindung detailliert anhand der Zeichnungen beschrieben.

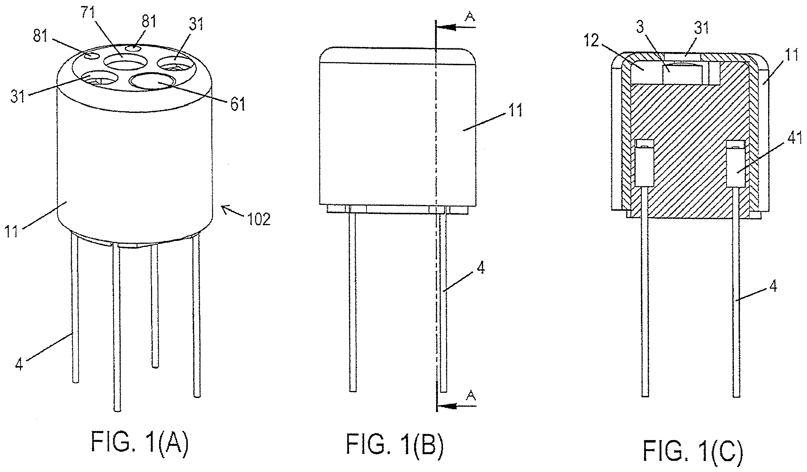

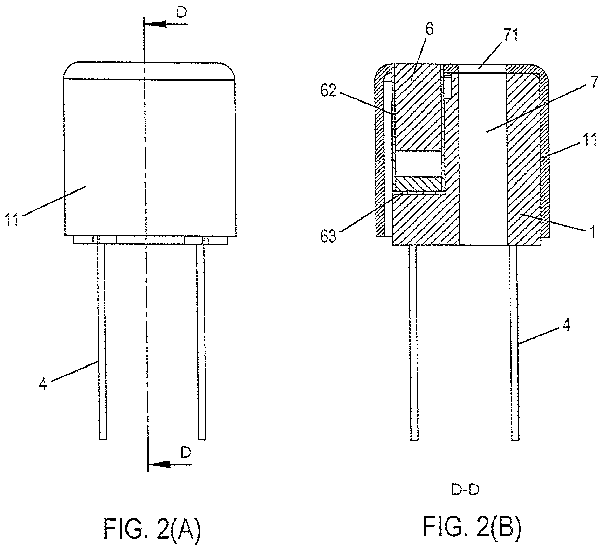

Figur 1 zeigt einen Endoskopkopf mit Zugseilen gemäß einem Ausführungsbeispiel der Erfindung, wobei Fig. 1(A) eine schematische perspektivische Ansicht, Fig. 1(B) eine Seitenansicht und Fig. 1(C) eine Schnittansicht entlang einer Linie A-A in Fig. 1(B) zeigen. Figur 2 zeigt den Endoskopkopf mit Zugseilen gemäß dem Ausführungsbeispiel von Figur 1, wobei Fig. 2(A) eine Seitenansicht und Fig. 2(B) eine Schnittansicht entlang einer Linie D-D in Fig. 2(A) zeigen.

Das dargestellte Endoskop weist an einem (nicht gezeigten) Katheterabschnitt einen Deflectingabschnitt (ebenfalls nicht gezeigt) auf. Der Deflectingabschnitt erstreckt sich von einem (nicht gezeigten) Ringelement an der proximalen Seite des Deflectingabschnittes zu einem Endoskopkopf 102 an der distalen Seite des Deflectingabschnittes. Der Endoskopkopf 102 ist somit an einem Deflectingende des Endoskops angeordnet.

Der Endoskopkopf 102 weist ein MID-Formelement (molded interconnected device) 1 als Endoskopkopfkörper auf. Das MID-Formelement 1 ist ein Kunststoffträger aus einem organischen polymeren Material, der z.B. durch Spritzgießen herstellbar ist. Beispielsweise besteht das MID-Formelement 1 aus einem Thermoplast oder einem Duroplast. Insbesondere kann das MID-Formelement 1 aus Polypropylen (PP), Acrylnitryl-Butadienstyrol (ABS), Polycarbonat (PC), Polyethylenterephtalat (PET), Polybutylenterephtalat (PBT), Polyamid (PA), Polyphenylensulfid (PPS), Polysulfon (PSU), Polyethersulfon (PES), Polyetherimid (PEI), etc hergestellt werden. Diese Angaben sind lediglich Beispiele und weitere Materialen können für das MID-Formelement 1 verwendet werden.

Das MID-Formelement 1 ist ein zylinderartiges Element, dessen Mittelachse sich von einer proximalen Seite zu einer distalen Seite erstreckt. An der proximalen Seite ist das MID-Formelement 1 an den Deflectingabschnitt verbindbar. Die distale Seite des MID-Formelementes 1 bildet die distale Seite des Endoskopkopfes 102.

An der distalen Seite und an den Seitenflächen ist das MID-Formelement 1 von einer Kappe 11 umgeben. Die Kappe 11 ist ein mit einem Boden versehener Hohlzylinder, wobei der Boden der Kappe im am MID-Formelement 1 aufgesetzten Zustand die distale Seite des Endoskopkopfes 102 bildet. Die Kappe 11 hat einen solchen Innendurchmesser, dass sie leichtgängig am Außendurchmesser des MID-Formelementes 1 sitzt. Alternativ kann auch eine Presspassung ausgebildet sein. In einer weiteren Alternative kann die Kappe 11 an der Innenumfangsfläche ein Innengewinde aufweisen und auf ein Außengewinde am MID-Formelement 1 aufgeschraubt werden. Weitere formschlüssige Verbindungen zwischen Kappe 11 und MID-Formelement 1 sind denkbar.

An der distalen Stirnfläche besitzt die Kappe 11 distale Öffnungen 31 für eine Signalausgabe eines nachstehend beschriebenen LED-Chips 3, eine distale Öffnung für ein Kamerafenster 61 für eine Signaleingabe für eine nachstehend beschriebene Kamera 6, eine distale Arbeitskanalöffnung 71 und distale Spülkanalöffnungen 71.

Im am MID-Formelement 1 aufgesetzten Zustand der Kappe 11 ragt das MID-Formelement 1 an der proximalen Seite über die Kappe 11 vor. An dem aus der Kappe 11 proximal vorragenden Außenumfang des MID-Formelementes 1 wird ein distaler Endabschnitt eines nicht gezeigten Schlauchelementes des Deflectingabschnittes aufgesetzt. Die distale Stirnfläche des Schlauchelementes liegt an der proximalen Endfläche der Kappe 11 abgedichtet an. Die distale Stirnfläche des Schlauchelementes kann mit der proximalen Endfläche der Kappe 11 verklebt sein.

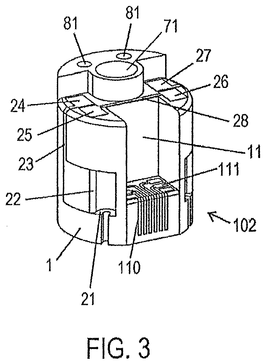

Figur 3 zeigt das MID-Formelement 1 als den Endoskopkopfkörper für den Endoskopkopf gemäß dem Ausführungsbeispiel von Figur 1.

Das MID-Formelement 1 ist mit einer oder mehreren metallischen Leiterbahnen 21-28, 110, 111 versehen. Die Leiterbahnen 21-28, 110, 111 können am MID-Formelement 1 durch Zweikomponentenspritzguss, durch Heißprägen, ein Maskenbelichtungsverfahren, durch Laserstrukturierverfahren, durch Folienhinterspritzen oder ein anderes geeignetes Verfahren aufgetragen werden. Im Prinzip bildet das mit der/den Leiterbahnen 21-28, 110, 111 versehene MID-Formelement 1 eine dreidimensionale Leiterplatte.

Genauer gesagt sind am MID-Formelement 1 Leiterbahnen 21-28 für nachstehend beschriebene LED-Chips 3 und Leiterbahnen 110, 111 für ein Kameramodul 6 angeformt.

Nachstehend sind die Leiterbahnen genauer beschrieben.

Unter Betrachtung von Figur 3 besitzt das als dreidimensionale Leiterplatte ausgebildete MID-Formelement 1 mehrere Ebenen, die als erste Ebene für einen Zugseilverankerungskörperanschluss, zweite Ebene für einen Kameraanschluss, dritte Ebene für einen LED-Anschluss und vierte Ebene als distale Endfläche bezeichnet werden können. Die erste Ebene und die zweite Ebene können zueinander versetzt sein oder auf gleicher Höhe liegen.

Beabstandet zur proximalen Endfläche besitzt das MID-Formelement 1 an seiner Außenumfangsfläche jeweilige Einbauchungen für nachstehend beschriebene Zugseilverankerungskörper 41. Jede dieser Einbauchungen hat eine derartige Größe, dass ein Zugseilverankerungskörper 41 darin Platz findet, besitzt an ihrer proximalen Seite einen Abstützflächenbereich, an dem sich der Zugseilverankerungskörper 41 in proximaler Richtung abstützen kann. Der Abstützflächenbereich erstreckt sich somit horizontal d.h. parallel zur proximalen Endfläche des MID-Formelements 1 und befindet sich an der vorstehend beschriebenen ersten Ebene. Die Einbauchungen sind an die Form des Zugseilverankerungskörpers 41 angepasst und sind im vorliegenden Beispiel zylinderartig gestaltet. Somit hat im vorliegenden Beispiel das MID-Formelement 1 vier solche Abstützflächenbereiche. Jeder Abstützflächenbereich ist zentrisch mit einer zur proximalen Endfläche des MID-Formelements 1 laufenden rinnenartigen Einkerbung versehen, die bei am Abstützflächenbereich eingesetzten Zugseilverankerungskörper 41 das mit dem Zugseilverankerungskörper 41 verbundene Zugseil 4 aufnimmt. Der Durchmesser des Abstützflächenbereiches ist auf jeden Fall größer als der Durchmesser des Zugseils 4. Sollte z.B. ein quaderartiger Zugseilverankerungskörper angewendet werden und die Einbauchung daher eine quaderartige Form haben, ergibt sich ein viereckiger Abstützflächenbereich.

Zumindest für zwei Zugseilverankerungskörper (in Figur 3 an den beiden dem Betrachter zugewandten Einbauchungen) ist der Abstützflächenbereich mit einer ersten Leiterbahn 21 ausgeformt. Mit der Leiterbahn 21 ist jeweils eine sich zunächst senkrecht nach oben erstreckende zweite Leiterbahn 22 verbunden, die am Rand der Einbauchung angeformt ist und sich bei Erreichen des oberen Randes der Einbauchung horizontal von der Einbauchung weg erstreckt und schließlich, wie in Figur 3 gezeigt, in eine sich senkrecht nach oben erstreckende dritte Leiterbahn 23 übergeht. Die dritte Leiterbahn 23 ist mit einem flächigen Leiterbahnabschnitt 24 verbunden, der an der dritten Ebene des MID-Formelements 1 angeformt ist. Der flächige Leiterbahnabschnitt 24 bildet eine Anode für einen ersten LED-Chip 3. Benachbart und kontaktfrei zum flächige Leiterbahnabschnitt 24 ist ein flächiger Leiterbahnabschnitt 25 an der dritten Ebene des MID-Formelements 1 angeformt. Der flächige Leiterbahnabschnitt 25 bildet eine Kathode für den ersten LED-Chip 3. Die Leiterbahneabschnitte 21-25 befinden sich unter Betrachtung von Figur 3 an der linken Seite eines oberhalb der zweiten Ebene für einen Kameraanschluss vorgesehenen Kameraunterbringungsraums. An der unter Betrachtung von Figur 3 rechten Seite des Kameraunterbringungsraums ist eine ähnliche Leiterbahnstruktur wie an der linken Seite ausgebildet. In Figur 3 ist rechts neben der zweiten Ebene für einen Kameraanschluss eine Einbauchung für einen Zugseilverankerungskörper erkennbar, die ebenfalls mit einer Leiterbahn wie die erste Leiterbahn 21 ausgebildet ist. Diese Leiterbahn ist mit einem flächigen Leiterbahnabschnitt 26 an der dritten Ebene verbunden. Der flächige Leiterbahnabschnitt 26 bildet eine Kathode für einen zweiten LED-Chip 3. Benachbart und kontaktfrei zum flächigen Leiterbahnabschnitt 26 ist ein flächiger Leiterbahnabschnitt 27 an der dritten Ebene des MID-Formelements 1 angeformt. Der flächige Leiterbahnabschnitt 27 bildet eine Anode für den zweiten LED-Chip 3. Der flächige Leiterbahnabschnitt 25 ist über einen Steg 28 mit dem flächigen Leiterbahnabschnitt 26 verbunden, wie dies in Figur 3 gezeigt ist. Der flächige Leiterbahnabschnitt 27 ist mit dem flächigen Leiterbahnabschnitt 24 verbunden, was in der Darstellung von Figur 3 nicht gezeigt ist.

An der proximalen Seite des Kameraunterbringungsraums, d.h. auf der zweiten Ebene des MID-Formelements 1 sind Leiterbahnabschnitte 110 ausgeformt, die in flächige Leiterbahnabschnitte 111 für einen Anschluss eines Kameramoduls 6 münden. Die flächigen Leiterbahnabschnitte 111 sind auch auf der zweiten Ebene des MID-Formelements 1 ausgeformt. Die Leiterbahnabschnitte 110 sind mit einem (nicht gezeigten) Kamerakabelanschluss elektrisch verbunden, der an der proximalen Fläche des MID-Formelements 1 vorgesehen ist. An diesem Kamerakabelanschluss ist ein Versorgungskabel eingesteckt, dass im Deflectingabschnitt geführt ist.

Außerdem weist das MID-Formelement 1 an der vierten Ebene eine distale Arbeitskanalöffnung 71 als Verlängerung eines nicht gezeigten Arbeitskanalelementes für z.B. Mikrowerkzeuge und zumindest eine distale Spülkanalöffnung 81 zumindest eines Spülkanals auf. Im vorliegenden Beispiel weist das MID-Formelement 1 zwei distale Spülkanalöffnungen 81 für zwei Spülkanale auf. Genauer gesagt sind im MID-Formelement 1 in axialer Richtung parallel zur Mittelachse des MID-Formelementes 1 in dem Bereich, der nicht von dem Kameraunterbringungsraum und dem Leiterbahnenbereich eingenommen wird, ein Arbeitskanalendabschnitt und zwei Spülkanalendabschnitte als Durchgangslöcher vorgesehen. Wie in Fig. 1 gezeigt, ist der Arbeitskanalendabschnitt zwischen den beiden Spülkanalendabschnitten benachbart angeordnet. An der distalen Stirnfläche des MID-Formelementes 1 hat der Arbeitskanalendabschnitt die distale Arbeitskanalöffnung 71 und haben die beiden Spülkanalendabschnitte jeweils eine distale Spülkanalöffnung 81. Der Arbeitskanalendabschnitt ist dabei parallel und benachbart zu dem Kameraunterbringungsraum angeordnet. Die Spülkanalendabschnitte sind radial außerhalb des Arbeitskanalendabschnitt angeordnet, wobei die Erfindung nicht darauf beschränkt ist.

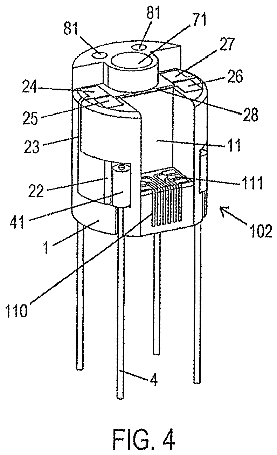

Nachstehend ist der weitere Aufbau des Endoskopkopfes 102 detailliert erläutert. Figur 4 zeigt einen Endoskopkopfkörper von Figur 3, in den Zugseile eingehängt sind.

In jede seitliche Einbauchung am MID-Formelement 1 ist ein Zugseilverankerungskörper 41 so eingesetzt, dass seine proximalen Seite am Abstützflächenbereich anliegt, so dass eine in proximaler Richtung wirkende Zugkraft vom Zugseilverankerungskörper 41 auf den Abstützflächenbereich und somit auf das MID-Formelement 1 übertragen wird. Jeder Zugseilverankerungskörper 41 ist am distalen Ende eines Zugseils 4 in bekannter Weise fest angeordnet.

Im vorliegenden Beispiel sind zwei seitliche Einbauchung am MID-Formelement 1 wie vorstehend beschrieben mit Leiterbahnen versehen. In diese Einbauchungen ist jeweils ein Zugseilverankerungskörper 41 mit Zugseil 4 angeordnet, die elektrisch leitfähig sind. Im vorliegenden Beispiel sind zwei der vier Abstützflächenbereiche mit jeweils einem Leiterbahnabschnitt ausgekleidet.

Die beiden in Figur 4 vorderen Zugseilverankerungskörper 41, die jeweils an einem Leiterbahnabschnitt 2 sitzen, und die mit ihnen verbundenen Zugseile 4 sind elektrisch leitfähig. Jeder Leiterbahnabschnitt 2, an dem ein elektrisch leitfähiger Zugseilverankerungskörper 41 sitzt, bildet einen elektrischen Anschluss für ein elektronisches Instrument. Somit können die Leiterbahnabschnitte 21-28 über die elektrisch leitfähigen Zugseile 4 mit elektrischem Strom versorgt werden. Eine Gleichspannungsversorgung oder eine Wechselspannungsversorgung des elektronischen Instruments oder der elektronischen Instrumente sind dadurch möglich. Im vorliegenden Beispiel von Fig. 1 sind am MID-Formelement vier Zugseile 4 verankert, von denen zwei Zugseile 4 mit der zumindest einen Leiterbahn 2 des MID-Formelementes 1 elektrisch in Verbindung stehen.

Ein solcher Zugseilverankerungskörper 41 kann verschiedene Formen haben und kann ein Tonnennippel, ein Birnennippel, ein Kugelnippel etc. sein.

Im vorliegenden Beispiel hat das MID-Formelement 1 vier Abstützflächenbereiche für vier Zugseilverankerungskörper 41. Die Erfindung ist nicht darauf beschränkt. Der Endoskopkopf 102 kann drei, fünf oder mehr Zugseilverankerungskörper 41 und in entsprechender Anzahl Abstützflächenbereiche am MID-Formelement 1 aufweisen.

Der Endoskopkopf 102 kann anhand einer Zugbewegung an den Zugseilen 4 durch ein nicht gezeigtes Steuerelement in eine beliebige Richtung in bekannter Weise geschwenkt werden.

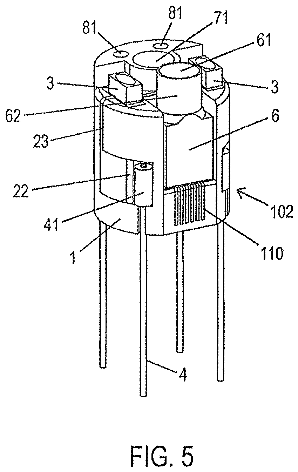

Figur 5 zeigt einen Endoskopkopfkörper von Figur 4, in den ein Kameramodul und LED-Chips eingesetzt ist.

Auf dem flächigen Leiterbahnabschnitt 24 (Anode) und dem flächigen Leiterbahnabschnitt 25 (Kathode) ist ein erster LED-Chip 3 angeordnet. Auf dem flächigen Leiterbahnabschnitt 26 (Kathode) und dem flächigen Leiterbahnabschnitt 27 (Anode) ist ein zweiter LED-Chip 3 angeordnet. Insbesondere sind die LED-Chips 3 so angeordnet, dass ihre Anschlüsse an der proximalen Seite den flächigen Leiterbahnabschnitten 24-27 zugewandt sind. Die Abgabefläche der LED-Chips 3 befindet sich an deren distaler Seite. Die Abgabefläche der LED-Chips 3 befindet sich auf einer Ebene die parallel und beabstandet zur distalen Endfläche (vierte Ebene) des MID-Formelementes 1 ist. Anders ausgedrückt ragt die zur distalen Endfläche (vierte Ebene) des MID-Formelementes 1 in distaler Richtung über die Abgabefläche der LED-Chips 3 vor.

In den Kameraunterbringungsraum ist das Kameramodul 6 so angeordnet, dass die Anschlusskontakte 63 des Kameramoduls 6 mit den flächigen Leiterbahnabschnitten 111 in Kontakt stehen, siehe auch Figur 2 (B). Insbesondere ist das Kameramodul 6 so angeordnet, dass seine Anschlusskontakte an der proximalen Seite den flächigen Leiterbahnabschnitten 111 zugewandt sind. An der distalen Seite ist ein Kamerafenster 61 angeordnet, über das Bildinformationen aufgenommen werden können. Der distale Endbereich des Körpers des Kameramoduls 6 erstreckt sich bis zu dem Kamerafenster 61 und ist von einer zylindrischen Abschirmung 62 umgeben.

Die Abschirmung 62 erstreckt sich in distaler Richtung bis über die distale Endfläche des MID-Formelementes 1 hinaus und ragt also von der vierten Ebene des MID-Formelementes 1 geringfügig vor. Die Abschirmung 62 erstreckt sich somit in distaler Richtung bis über die die horizontale Ebene der Abgabefläche der LED-Chips 3. Dadurch wird durch die Abschirmung 62 eine Abschirmung des Kameramoduls 6 vor der Lichtstrahlung der LED-Chips 3 geschaffen.

Die Wandstärke der distalen Endfläche der Kappe 11 ist so gewählt, dass sie der Höhendifferenz zwischen der vierten Ebene des MID-Formelementes 1 und der distalen Endseite der von der vierten Ebene des MID-Formelementes 1 vorragenden Abschirmung 62 entspricht. Somit liegt bei auf dem MID-Formelement 1 aufgesetzter Kappe 11 die proximale Innenfläche der distalen Endfläche der Kappe 11 an der vierten Ebene des MID-Formelementes 1 an, und die distale Endseite der Abschirmung 62 ist in etwas fluchtend zur distalen Außenfläche der distalen Endfläche der Kappe 11.

Zwischen der proximalen Innenfläche der distalen Endfläche der Kappe 11 und der dritten Ebene des MID-Formelementes 1, d.h. der Ebene für den LED-Anschluss, ist daher ein Hohlraum 12 gebildet. Dieser Hohlraum 12 ist an der Oberseite durch die Kappe 11 begrenzt und (bei aufgesetzter Kappe 11) nur an den Öffnungen 31 an der distalen Seite offen, siehe Figur 1(C). Die Öffnungen 31 sind jeweils genau oberhalb der LED-Chips 3 angeordnet.

Der Hohlraum 12 kann (aber muß nicht) mit einer Vergussmasse ausgefüllt sein, die die Leiterbahnabschnitte und LED-Chips 3 bedeckt. Die Vergussmasse ist transparent und aushärtbar und besitzt ein ausreichendes Haftungsvermögen für ein Anhaften am Boden und den Wänden des Hohlraums 12.

### Verfahren zum Herstellen des MID-Formelementes

Das MID-Formelement 1 mit der/den Leiterbahnen als dreidimensionale Leiterplatte kann auf verschiedene Weise hergestellt werden.

Beispielsweise kann ein Zweistufenformverfahren angewendet werden.

Zunächst wird ein Kunststoffträger als Grundkörper des MID-Formelementes 1 spritzgegossen. Danach werden die Leiterbahn(en) auf dem Grundkörper z.B. durch Zweikomponentenspritzguss, Heißprägen, Maskenbelichtung oder Laserstrukturierung etc. aufgetragen.

Die Formgebung ist dabei weitgehend frei wählbar. Als Endoskopkopfkörper wird das MID-Formelement 1 zylinderartig mit einem kleinen Außendurchmesser so gestaltet, dass ausreichend Platz für den Arbeitskanal und den/die Spülkanäle belassen bleibt.

### Weitere Alternativen

Im vorliegenden Beispiel der Figuren 1-5 sind am MID-Formelement 1 vier Zugseile 4 verankert, von denen zwei Zugseile 4 mit der zumindest einen Leiterbahn 2 des MID-Formelementes 1 elektrisch in Verbindung stehen. Die Erfindung ist aber auch auf ein MID-Formelement 1 anwendbar, bei dem kein Zugseil 4 elektrisch leitfähig ist. Die elektrische Versorgung der Leiterbahn 2 wird dann über im Deflectingabschnitt 100 verlaufende Kabel bewerkstelligt.

Im vorliegenden Beispiel ist die Kappe 11 auf das MID-Formelement 1 aufgesetzt. Die Kappe 11 kann auch weggelassen werden. In diesem Fall wird die Außenfläche und die distale Stirnfläche des MID-Formelement 1 so geformt, dass sie keine Hinterschneidungen aufweist und daher gut gereinigt werden kann.

Im vorliegenden Beispiel der Figuren 1-5 liegt die Außenfläche des Kameramoduls 6 an der Innenfläche der Kappe 11 an. Das MID-Formelement 1 kann auch so geformt sein, dass im MID-Formelement 1 eine spezifische Kameravertiefung mit distaler Öffnung vorgesehen ist, in die das Kameramodul 6 von der distalen Seite eingeschoben wird. Die Seitenflächen des Kameramoduls 6 sind dann mit Ausnahme an der distalen Seite von dem MID-Formelement 1 umgeben. Die spezifische Kameravertiefung kann passgenau für das Kameramodul 6 bemessen sein. Die an der distalen Seite von dem MID-Formelement 1 vorragende Kameraabschirmung 62 kann an der proximalen Seite im MID-Formelement 1 einrastbar sein, um dem Kameramodul 6 im MID-Formelement 1 sicheren Halt zu geben und gleichzeitig leicht entnehmbar zu sein. Alternativ ist das Kameramodul 6 am MID-Formelement 1 durch eine Vergussmasse fest angegossen.

Bei weggelassener Kappe 11 kann das MID-Formelement 1 so ausgebildet sein, dass es eine umlaufende Wand bis zu der Höhe der vierten Ebene hat. Dann bildet die Vergussmasse an der distalen Seite eine Endfläche, die die distale Endfläche des Endoskopkopfes bildet. Die distale Endfläche der Vergussmasse ist nach innen gewölbt ausgebildet kann aber in einem weiteren Bespiel auch flach d.h. eben sein. Die Deckfläche der transparenten und ausgehärteten Vergussmasse an der distalen Seite des MID-Formelementes ist also nach innen gewölbt oder erstreckt sich plan. Auch eine nach außen gerichtete Wölbung der distalen Endfläche der Vergussmasse ist möglich. Am distalen Ende des MID-Formelementes 1 ist somit ebenfalls ein Raum wie der Hohlraum 12 vorhanden, in dem zumindest ein elektronisches Instrument 3 auf einer Leiterbahn 2 des MID-Formelementes 1 sitzt, wobei dieser Raum durch die transparente und ausgehärtete Vergussmasse gefüllt ist. Die Abschirmung erstreckt sich auch in diesem Fall in distaler Richtung bis über die distale Endfläche des MID-Formelementes 1 und ragt von der vierte Ebene des MID-Formelementes 1 der distalen Endfläche der Vergussmasse geringfügig vor.

Im MID-Formelement 1 erstreckt sich ein Arbeitskanalabschnitt und zwei Spülkanalabschnitte parallel und beabstandet zur Mittelachse des MID-Formelementes 1. Der Arbeitskanalabschnitt kann auch auf der Mittelachse des MID-Formelementes 1 angeordnet sein. Es können drei oder mehr Spülkanäle vorgesehen sein. Jedoch ist des kleines Aufbaus wegen eine Konstruktion mit einem oder zwei Spülkanälen zu bevorzugen. Der Arbeitskanalabschnitt und/oder die Spülkanäle kann/können in einer weiteren Alternative im MID-Formelement 1 schräg verlaufen.

Im vorliegenden Beispiel ist ein Kameramodul 6 als ein optischer Sensor am MID-Formelement 1 angeordnet, und sind LED-Chips 3 als optische Signalgeber als ein Beispiel für ein elektronisches Instrument vorgesehen. Alternativ kann anstelle der LED-Chips 3 als elektronisches Instrument eine Ultraschallemittiereinrichtung sein, und anstelle des Kameramoduls 6 kann ein Akustiksensor angeordnet sein, wobei der Akustiksensor zum Hohlraum hin abgeschirmt ist.

Wenn der Sensorbereich für eine andere Sensorart genutzt wird, wie z.B. ein akustischer Sensor, können die von der distalen Endfläche der Vergussmasse vorragenden Abschirmungswandabschnitte auch weggelassen werden.

### Bezugszeichenliste

1 MID-Formelement
11 Kappe
12 Hohlraum
21-28, 110, 111 Leiterbahn
3 LED-Chip
31 Öffnung
4 Zugseil
41 Zugseilverankerungskörper
6 Kameramodul
61 Kamerafenster
62 Kameraabschirmung
63 Kameraanschlusskontakte
7 Arbeitskanalelement
71 distale Arbeitskanalöffnung
8 Spülkanal
81 distale Spülkanalöffnung
102 Endoskopkopf
110 Leiterbahnabschnitte
111 Leiterbahnabschnitte für Anschluss des Kameramoduls

## Patentansprüche

1. Endoskopkopf (102) an einem Deflectingende eines Endoskops, mit
einem Endoskopkopfkörper (1) mit zumindest einer an diesem aufgetragenen Leiterbahn (21-28);
zumindest einem elektronischen Instrument (3), das im Endoskopkopfkörper (1) sitzt und durch dessen zumindest eine Leiterbahn (21-28) elektrisch versorgbar ist; und
zumindest einem Zugseil (4), dessen Zugseilverankerung (41) im Endoskopkopfkörper (1) sitzt, wobei der Endoskopkopf (102) so aufgebaut ist, dass der Endoskopkopf (102) anhand einer Zugbewegung am Zugseil (4) geschwenkt werden kann;
wobei das zumindest eine Zugseil (4) mit der zumindest einen Leiterbahn (21-28) des Endoskopkopfkörpers (1) elektrisch in Verbindung steht.

2. Endoskopkopf (102) gemäß Anspruch 1,
das zumindest eine Zugseil (4) über seine Zugseilverankerung (41) mit der zumindest einen Leiterbahn (21-28) des Endoskopkopfkörpers (1) elektrisch in Verbindung steht.

3. Endoskopkopf (102) gemäß Anspruch 1 oder 2, wobei
am Endoskopkopfkörper (1) vier Zugseile (4) verankert sind, von denen zwei Zugseile mit der zumindest einen Leiterbahn (21-28) des Endoskopkopfkörpers (1) elektrisch in Verbindung stehen.

4. Endoskopkopf (102) gemäß einem der Ansprüche 1 bis 3, wobei
der Endoskopkopfkörper ein MID-Formelement (1) ist.

5. Endoskop mit einem Endoskopkopf (102) gemäß einem der Ansprüche 1 bis 4.

## Claims

1. An endoscope head (102) on a deflecting end of an endoscope, comprising
an endoscope head body (1) having at least one conducting path (21-28) applied thereon;
at least one electronic instrument (3), which is seated in the endoscope head body (1) and can be electrically supplied by said at least one conducting path (21-28); and
at least one pulling cable (4) having an anchoring (41) which is seated in the endoscope head body (1), wherein the endoscope head (102) is structured such that the endoscope head (102) can be pivoted with the aid of a pulling movement on the pulling cable (4);
wherein the at least one pulling cable (4) is electrically connected to the at least one conducting path (21-28) of the endoscope head body (1).

2. The endoscope head (102) according to claim 1, [wherein]
the at least one pulling cable (4) is electrically connected via its anchoring (41) to the at least one conducting path (21-28) of the endoscope head body (1).

3. The endoscope head (102) according to claim 1 or 2, wherein
four pulling cables (4) are anchored on the endoscope head body (1), of which two pulling cables are electrically connected to the at least one conducting path (21-28) of the endoscope head body (1).

4. The endoscope head (102) according to any of claims 1 to 3, wherein
the endoscope head body is an MID molded interconnect element (1).

5. An endoscope having an endoscope head (102) according to any of claims 1 to 4.

## Revendications

1. Tête d'endoscope (102) sur une extrémité déflectrice, avec
un corps de tête d'endoscope (1) avec au moins un tracé conducteur (21-28) appliqué sur celui-ci ;
au moins un instrument électronique (3), qui repose dans le corps de tête d'endoscope (1) et peut être alimenté électriquement par le au moins un tracé conducteur (21-28) de celui-ci ; et
au moins un câble de traction (4), dont l'ancrage de câble de traction (41) repose dans le corps de tête d'endoscope (1), dans laquelle la tête d'endoscope (102) est conçue de sorte que la tête d'endoscope (102) peut être amenée à pivoter sur le câble de traction (4) à l'aide d'un mouvement de traction ;
dans laquelle le au moins un câble de traction (4) est relié électriquement à le au moins un tracé conducteur (21-28) du corps de tête d'endoscope (1).

2. Tête d'endoscope (102) selon la revendication 1, [dans laquelle]
le au moins un câble de traction (4) est relié électriquement à le au moins un tracé conducteur (21-28) du corps de tête d'endoscope (1) par l'intermédiaire de son ancrage de câble de traction (41).

3. Tête d'endoscope (102) selon la revendication 1 ou 2, dans laquelle
quatre câbles de traction (4), dont deux câbles de traction sont reliés électriquement à le au moins un tracé conducteur (21-28) du corps de tête d'endoscope (1), sont ancrés sur le corps de tête d'endoscope (1).

4. Tête d'endoscope (102) selon l'une quelconque des revendications 1 à 3, dans laquelle
le corps de tête d'endoscope est un élément moulé MID (1).

5. Endoscope avec une tête d'endoscope (102) selon l'une quelconque des revendications 1 à 4.
